Europäisches Patentamt

European Patent Office

Office européen des brevets

(19)

(11) Veröffentlichungsnummer: **0 069 343**
**B1**

(12) **EUROPÄISCHE PATENTSCHRIFT**

(45) Veröffentlichungstag der Patentschrift:
14.11.84

(51) Int. Cl.³: **C 07 C 93/00, C 07 D 295/08**

(21) Anmeldenummer: **82105857.5**

(22) Anmeldetag: **01.07.82**

(54) Quaternäre Ammoniumsalze und Verfahren zu ihrer Herstellung.

(30) Priorität: **04.07.81 DE 3126522**

(43) Veröffentlichungstag der Anmeldung:
**12.01.83 Patentblatt 83/2**

(45) Bekanntmachung des Hinweises auf die Patenterteilung:
**14.11.84 Patentblatt 84/46**

(84) Benannte Vertragsstaaten:
**BE CH DE FR GB IT LI NL**

(73) Patentinhaber: **BASF Aktiengesellschaft,
Carl-Bosch-Strasse 38, D-6700 Ludwigshafen (DE)**

(72) Erfinder: **Strickler, Rainer, Dr., Schroederstrasse 14,
D-6900 Heidelberg (DE)**

(56) Entgegenhaltungen:
DE - A - 1 593 746
DE - B - 1 077 207
FR - A - 2 113 921
GB - A - 1 025 160
US - A - 2 049 463
US - A - 2 103 272
US - A - 3 448 134

CHEMICAL ABSTRACTS, Band 31, Nr. 4, 20. Februar
1937, Spalte 1102 -5, Columbus Ohio (USA); R. HUNT et
al.: "Ethers of choline and allied compounds"

# Beschreibung

In der Gruppe der kationischen Tenside haben die quaternären Ammoniumsalze ausschlaggebende Bedeutung erlangt. Sie finden Verwendung z.B. als Biozide, als kationaktive Detergentien, als Emulgatoren im Bauten- und Korrosionsschutz sowie bei der Erzaufbereitung und als Färbereihilfsmittel in der Textil- und Lederindustrie.

Ausgangsprodukte für quaternäre (quartäre) Ammoniumsalze sind tertiäre Amine, die durch Alkylierung in die Ammoniumsalze übergeführt werden können. Ihre Verfügbarkeit ist also von der Zugänglichkeit der entsprechenden tertiären Amine abhängig.

Tertiäre Amine werden bisher im wesentlichen durch katalytische hydrierende Alkylierung von Amin/Alkohol- bzw. Amin/Aldehyd-Gemischen hergestellt. Typische Nebenreaktionen dieser Umsetzung sind Umalkylierungen. Sie führen vor allem bei unsymmetrisch substituierten Aminen zu Produktgemischen, die nur schwer aufgearbeitet werden können. Vor allem bei der Umsetzung von Polyalkylenglykolethern als Alkoholkomponente mit Aminen unter den Bedingungen der alkylierenden Hydrierung fallen durch Etherspaltung Produktgemische an. So ist es sehr schwierig, tertiäre Alkyl- bzw. Arylpolyetheramine bestimmter Struktur in guter Ausbeute herzustellen und folgerichtig sind auch entsprechende quaternäre Ammoniumsalze schwer zugänglich.

(Poly)alkanolamine mit freien Hydroxylgruppen und deren Ammoniumsalze sind dagegen leicht zugänglich und zwar über die Oxalkylierung von Ammoniak bzw. primären oder sekundären Aminen mit Alkylenoxiden, wobei nach bekannten Methoden mit Alkylierungsmitteln quaternäre Ammoniumsalze erhalten werden, die ebenso wie die eingesetzten Alkanolamine noch Hydroxylgruppen enthalten. Diese quaternierten Alkanolamine bleiben nun bei vielen Anwendungszwecken den einfachen Polyalkylammoniumsalzen unterlegen.

Eine Möglichkeit, unter Veretherung der Hydroxylgruppen dieser Alkanolammoniumsalze bei guten Ausbeuten zu endgruppenverschlossenen Polyetherquartärsalzen zu gelangen, bestand bisher nicht. Unter den Bedingungen der Williamson-Ether-Synthese aus Alkoholat und Alkylierungsmittel, z.B. Alkylhalogenid, zersetzen sich die Quartärsalze unter Hofmann-Abbau.

In der US-A Nr. 2103272 wird ein Verfahren zur Herstellung von veretherten β-Methylcholinhalogeniden beschrieben, wobei zunächst in einer Williamson-Synthese der Ether hergestellt wird und anschliessend die N-Alkylierung erfolgt.

Nach dem Vorschlag der US-A Nrn. 2049463 und 3448134 können veretherte (β-Alkyl)cholinhalogenide durch Umsetzung von Trimethylamin mit den jeweiligen Halogenethylderivaten erhalten werden.

In der FR-A Nr. 7140410 werden quaternäre Ammoniumsalze beschrieben, die sich von Tris(2-propyloxyethyl)amin ableiten.

Die GB-A Nr. 1025160 offenbart N-Benzylammoniumsalze, die sich von Alkylethern von Alkanolaminen ableiten.

Aus der DE-A Nr. 1593746 ist die Herstellung von langkettigen N-Alkylammoniumsalzen bekannt, die sich ebenfalls von Alkylethern von Alkanolaminen ableiten.

In der DE-A Nr. 1077207 wird vorgeschlagen, Vinylether, die eine tertiäre Aminogruppe enthalten, am Stickstoffatom zu alkylieren.

Schliesslich werden in „Chem. Abstr.", *31*, 1102[5] (1937), Halogenide von Alkyl-, Aryl- und Benzylethern von Cholin bzw. von 2-Diethylaminoethanol beschrieben.

Es wurden nun neue quaternäre Ammoniumsalze der allgemeinen Formel

$$[R^1R^2R^3N(A-O)_n-R^3]^+Z^-$$

gefunden, wobei $R^1$ und $R^2$ jeweils gleiche oder verschiedene Substituenten aus der Gruppe der Alkyl-, Aryl-, Aralkyl-, Alkylaryl-, Alkoxyalkyl- oder (Poly)alkylenglykolreste bedeuten oder zusammen mit dem Stickstoffatom einen entsprechenden heterocyclischen Ring bilden oder wobei mindestens ein Substituent $R^1$ oder $R^2$ mehrfunktionell derart ist, dass ein wenigstens 2 Ammoniumgruppen aufweisendes Salz dargestellt wird; wobei $R^3$ einen Benzylrest, A einen Alkylenrest mit 2 bis 10 C-Atomen, n eine ganze Zahl von 1 oder mehr und Z entweder den Substituenten Chlor oder ein anderes salzbildendes Anion oder ein Hydroxylion bedeuten.

Die Alkylierung von Alkanolaminen (I) bzw. Alkanolammoniumsalzen (II) mit Benzylchlorid ($R^3Z$) in Gegenwart von Phasentransferkatalysatoren liefert bei milden Bedingungen quantitativ die neuen Stoffe, und zwar endgruppenverschlossene Polyetherquartärsalze (III) nach dem Schema

$$R^1R^2N(-A-O)_nH$$
$$(I)$$
$$+ R^3Z \xrightarrow{NaOH} [R^1R^2R^3N(-A-O)_nH]^+Z^-$$
$$(II)$$

$$[R^1R^2R^3N(-A-O)_nH]^+Z^-$$
$$(II)$$
$$+ R^3X \xrightarrow[NaOH]{Phasentransfer\ kat.}$$
$$[R^1R^2R^3N(-A-O)_n-R^3]^+Z^-$$
$$(III)$$

Ersichtlich kann man die erste Umsetzung als N-Alkylierung und die zweite als O-Alkylierung ansehen.

Die Substituenten $R^1$ und $R^2$ können dabei gleiche oder verschiedene Alkyl-, Cycloalkyl-, Aryl-, Aralkyl-, Alkylaryl-, Alkoxyalkyl- oder (Poly)alkylenglykolreste bedeuten; $R^1$ und $R^2$ können zusammen mit dem Stickstoffatom auch einen heterocyclischen Ring bilden, der weitere Heteroatome enthalten kann; $R^1$ und $R^2$ bedeuten in diesem Falle somit gemeinsam einen zweiwertigen Alkylen-, Oxaalkylen- oder Azaalkylenrest, der je nach Verfügbarkeit auch eine oder mehrere i.a. kurzkettige Alkylsubstituenten aufweisen kann.

Ebenso kann das neue Quartärsalz mehrere Ammoniumgruppen aufweisen, wenn man ausgeht von mehrfunktionellen Aminen, bei denen somit wenigstens ein Substituent $R^1$ bzw. $R^2$ wiederum durch eine tertiäre Aminogruppe $-NR^1R^2$ substituiert ist.

In den vorstehenden Formeln bedeutet ferner A einen Alkylenrest mit 2 bis 10 C-Atomen, $R^3$ einen Benzylrest und n eine ganze Zahl von 1 oder mehr, insbesondere 1 bis 5.

Z hat die Bedeutung von Chlor. Das Chloridion kann aber durch jedes beliebige andere anorganische oder organische Anion $Z^-$ ersetzt werden. Bevorzugt sind die Halogenide, Sulfate, Phosphate, Nitrate und Carboxylate.

An sich können die Substituenten $R^1$ und $R^2$ beliebige Form und Grösse haben, also z.B. geradkettig oder verzweigt sein; i.a. sind Alkylsubstitutenten (-gruppen) mit 1, insbesondere 2 bis 20, Arylgruppen mit 6 oder 10, Alkylaryl- und Aralkylgruppen mit 7 bis 20, Alkoxy(polyoxy)-alkylgruppen mit 3 bis 100 oder mehr und (Poly)-alkylenglykolgruppen mit 2 bis 100 oder mehr Kohlenstoffatomen von technischem Standpunkt aus besonders interessante Verbindungen.

Als Alkanolamine können demnach Umsetzungsprodukte von Ammoniak, primären oder sekundären Alkylaminen, mehrwertigen Aminen oder Polyaminen mit Alkylenoxiden, wie z.B. Dimethylethanolamin, Triethanolamin, Triisopropanolamin Butyldiethanolamin, Diethylethanolamin, Tetrahydroxiethylhexamethylendiamin und deren (Poly)oxalkylate, aber auch Alkanolamine anderer Herkunft, wie z.B. 3-Dimethylaminopropanol oder Dimethylneopentenolamin, N,N'-Dimethylneopentandiamin und deren Oxalkylate dienen. Als Alkylenoxid kommen Ethylenoxid, Propylenoxid oder ein Butylenoxid in Betracht. Die meisten der vorstehenden und viele andere solche Alkanolamine sind handelsüblich.

Als Alkylierungsmittel dient Benzylchlorid.

Als Base können grundsätzlich alle Alkali- und Erdalkalihydroxide dienen; aus wirtschaftlichen Gründen wird jedoch bevorzugt Natronlauge eingesetzt.

Als sog. Phasentransferkatalysatoren dienen quartäre Ammoniumsalze, wie sie in der einschlägigen Literatur beschrieben sind (vgl. E.V. Dehmlow, ,,Angew. Chemie" *86*, 187 bis 196 [1974]). Bekannt sind z.B. Trialkylbenzylammoniumsalze oder Tetraalkylammoniumsalze. Diese Ammoniumsalze können als Katalysatoren vor der Reaktion zugesetzt werden oder auch *in situ* im Zuge der Alkylierungsreaktion aus einem tertiären Amin hergestellt werden (z.B. Triethylbenzylammoniumchlorid aus Triethylamin und Benzylchlorid).

Zum Teil verlaufen die erfindungsgemässen Umsetzungen autokatalytisch, d.h. die entstehenden Quartärsalze katalysieren ihre eigene Veretherung, so dass sich die gesonderte Zugabe eines Phasentransferkatalysators erübrigt; dies lässt sich immer dadurch erreichen, dass ein Teil des umgesetzten Reaktionsgemisches zurückgeführt bzw.

als Lösungsmittel verwendet wird, vor allem bei kontinuierlichem (fortlaufendem) Betrieb.

Die Umsetzung kann einstufig, bevorzugt aber zweistufig durchgeführt werden. Erster Schritt ist dann die Quaternierung (N-Alkylierung) des vorgelegten Alkanolamins zum Alkanolammoniumsalz mit Benzylchlorid bei Temperaturen zwischen 20 und 160, bevorzugt zwischen 40 und 120°C; erst beim zweiten Schritt findet nach Zugabe von Alkali (bevorzugt als wässerige Lauge in Konzentrationen von 10 bis 60%) die phasentransferkatalysierte Veretherung (O-Alkylierung) zwischen 20 und 90, insbesondere zwischen 30 und 60°C statt. Bei einstufiger Umsetzung werden Alkanolamin und Lauge sowie gegebenenfalls der fremde Phasentransferkatalysator zusammen vorgelegt; das Benzylchlorid kann dann in einem Schritt oder im Mass der Umsetzung zugefügt werden. Der Quaternierungsschritt kann anhand der Aminzahl, der Veretherungsschritte, gegebenenfalls an der Freisetzung von Halogenid, aber auch an entnommenen Proben über die OH-Zahl verfolgt werden.

Die Umsetzung kann in ihrem eigenen Reaktionsgemisch, aber auch in Anwesenheit eines geeigneten Lösemittels, wie z.B. Benzol, Toluol, Chlorbenzol, Tetrachlorkohlenstoff, Methylenchlorid usw., bevorzugt jedoch in Wasser durchgeführt werden; gewöhnlich läuft sie bei atmosphärischem Druck ab.

Die Aufarbeitung des Reaktionsgemisches erfolgt durch Abtrennen der Lauge, gegebenenfalls nach Verdünnen mit einem Lösungsmittel, wie z.B. Isobutanol, Isopropanol oder anderer mit Lauge nicht oder nur begrenzt mischbarer Lösemittel. Zunächst erhält man durch Abdampfen des Lösemittels unter vermindertem Druck ein meist pastöses Rohprodukt. Dieses kann noch weiter gereinigt werden; so hat sich für eine Vielzahl der Produkte eine Umkristallisation aus Essigester oder Toluol bewährt, wobei weisse, kristalline Produkte mit definiertem Schmelzpunkt erhalten werden.

Besondere Vorteile des beschriebenen Verfahrens sind somit:

— Synthese der quaternären Ammoniumsalze, ausgehend von Aminen oder Ammoniak, in einem einzigen Reaktionsraum, z.B. einem Rührkessel oder einer Rührkesselkaskade;

— milde Reaktionsbedingungen;

— einfache Aufarbeitung ohne Destillationsschritte zur Trennung von Gemischen von Zielprodukten;

— gezielte Synthese definierter Produkte; kein Anfall von Isomerengemischen;

— Synthese quaternärer Ammoniumsalze ohne Abhängigkeit von schwer zugänglichen Trisalkylaminen.

Die erhaltenen Polyetherquartärsalze können zu den für quaternäre Ammoniumsalze üblichen Zwecken eingesetzt werden, z.B. als Textilhilfsmittel, kationaktive Tenside usw.

Die in der folgenden Tabelle als Beispiele angeführten einzelnen Stoffe sind sämtlich nach der nachstehenden Rahmenvorschrift erhältlich:

1 Eq tertiäres Alkanolamin und 0,03 Eq Benzyltriethylammoniumchlorid werden vorgelegt. Bei der erforderlichen Quaternierungstemperatur (s. Tabelle) wird 1,1 Eq des Alkylierungsmittels nach und nach zugesetzt und gerührt, bis der Amintiter kleiner als 0,1 mVal/g ist. Dann lässt man abkühlen, gibt unter weiterer Kühlung auf 40°C für n zu verethernde Mole Hydroxylgruppen (n+1) mol NaOH als 50%ige wässerige Lösung zu und setzt dann unter starker Rührung weitere n Mole Alkylierungsmittel zu. Bei 40°C rührt man einige Stunden nach; gegebenenfalls kann zwecks besserer Rührung mit Wasser verdünnt werden. Dann fügt man unter Rühren soviel Isobutanol und eine gleiche Menge Wasser zu, dass sich alles löst, rührt ½ h nach und lässt die wässerige Lauge ab. Man wäscht mit Wasser nach und destilliert schliesslich das Lösemittel unter vermindertem Druck ab. Das erhaltene pastöse Rohprodukt kann gegebenenfalls durch Eingiessen in 250 bis 500 ml Essigester ausgefällt und schliesslich abgesaugt werden.

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 1 | 89,1 g Dimethyl-ethanolamin (1 mol) 265,9 g Benzylchlorid (2,1 mol) 80 g NaOH | 100 | 12 | 281 | $(H_3C)_2\overset{\oplus}{N}-C_2H_4OCH_2\emptyset\ Cl^{\ominus}$ $\mid$ $CH_2$ $\emptyset$ |
| 2 | 117,2 g Diethylethanol-amin (1 mol) 265,9 g Benzylchlorid (2,1 mol) 80 g NaOH | 100 | 12 | 265 | $(H_5C_2)_2\overset{\oplus}{N}-C_2H_4OCH_2\emptyset\ Cl^{\ominus}$ $\mid$ $CH_2$ $\emptyset$ |
| 3 | 131,2 g 4-(2-Hydroxi-ethyl)morpholin (1 mol) 265,9 g Benzylchlorid (2,1 mol) 80 g NaOH | 100 | 12 | 196 | $O\begin{array}{c}CH_2-CH_2\\ \\CH_2-CH_2\end{array}\overset{\oplus}{N}C_2H_4OCH_2\emptyset\ Cl^{\ominus}$ $\mid$ $CH_2$ $\emptyset$ |
| 4 | 119,2 g N-Methyldi-ethanolamin (1 mol) 392,5 g Benzylchlorid (3,1 mol) 120 g NaOH | 100 | 12 | 360 | $H_3C\overset{\oplus}{N}(C_2H_4OCH_2\emptyset)_2\ Cl^{\ominus}$ $\mid$ $CH_2$ $\emptyset$ |
| 5 | 287,6 g N-Tridecyldi-ethanolamin (1 mol) 392,5 g Benzylchlorid (3,1 mol) 120 g NaOH | 100 | 12 | 568 | $H_{27}C_{13}\overset{\oplus}{N}(C_2H_4OCH_2\emptyset)_2\ Cl^{\ominus}$ $\mid$ $CH_2$ $\emptyset$ |
| 6 | 161,3 g N-n-Butyldi-ethanolamin (1 mol) 392,5 g Benzylchlorid (3,1 mol) 120 g NaOH | 100 | 24 | 450 | $n\text{-}H_9C_4\overset{\oplus}{N}-C_2H_4OCH_2\emptyset)_2\ Cl^{\ominus}$ $\mid$ $CH_2$ $\emptyset$ |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 7 | 149,2 g Triethanolamin (1 mol)<br>519,0 g Benzylchlorid (4,1 mol)<br>160 g NaOH | 110 | 20 | 364 | $\varnothing CH_2\overset{\oplus}{N}(C_2H_4OCH_2\varnothing)_3\ Cl^{\ominus}$ |

| Bei-spiel Nr. | Produkt | | | | | | | |
|---|---|---|---|---|---|---|---|---|
| | Summenformel | Elementaranalyse | | | | OHZ | Smp. (EE) (°C) | ¹H · NMR (Angaben in ppm) |
| | | C (%) | H (%) | N (%) | Cl⁻ (%) | | | |
| 1 | $C_{18}H_{24}NOCl$ | 70,1 (70,7) | 7,8 (7,8) | 4,2 (4,6) | 11,8 (11,6) | 13 (0) | 180 | in $CDCl_3$<br>$\delta = 3,3$ (s, 6H, N(CH₃)₂)<br>$\delta = 3,9$ (s, 4H, CH₂−CH₂)<br>$\delta = 4,5$ (s, 2H, ØCH₂O)<br>$\delta = 5,1$ (s, 2H, ØCH₂−N)<br>$\delta = 7,2$-7,8 (m, 10HØ) |
| 2 | $C_{20}H_{28}NOCl$ | 71,0 (72,0) | 8,5 (8,4) | 4,2 (4,2) | 10,1 (10,6) | 24 (0) | 116 | in $CDCl_3$<br>$\delta = 1,4$ (t, 6H, 2CH₃)<br>$\delta = 3,2$-4,1 (m, 8H, 4CH₂)<br>$\delta = 4,6$ (s, 2H, Ø−CH₂O)<br>$\delta = 4,9$ (s, 2H, ØOH₂N)<br>$\delta = 7,2$-7,7 (m, 10H, Ø) |
| 3 | $C_{20}H_{26}NO_2Cl$ | 68,7 (69,1) | 7,3 (7,5) | 4,2 (4,0) | 10,1 (10,2) | 8 (0) | 152 | in $CDCl_3$<br>$\delta = 3,3$-4,2 (m, 12H, 6CH₂)<br>$\delta = 4,6$ (s, 2H, ØCH₂O)<br>$\delta = 5,2$ (s, 2H, ØCH₂N)<br>$\delta = 7,1$-7,8 (m, 10H, Ø) |
| 4 | $C_{26}H_{32}NO_2Cl$ | 72,7 (73,3) | 7,4 (7,5) | 3,3 (3,3) | 8,5 (8,3) | 4 (0) | 107 | in $CDCl_3$<br>$\delta = 3,2$ (s, 3H, CH₃)<br>$\delta = 3,9$ (s, 8H, 2C₂H₄)<br>$\delta = 4,5$ (s, 4H, 2ØCH₂O)<br>$\delta = 5,1$ (s, 2H, ØCH₂N)<br>$\delta = 7,2$-7,5 (m, 15H, Ø) |
| 5 | $C_{38}H_{56}NO_2Cl$ | 76,9 (76,8) | 9,2 (9,4) | 2,3 (2,4) | 5,2 (6,0) | 34 (0) | | |
| 6 | $C_{29}H_{38}NO_2Cl$ | 73,8 (74,4) | 8,4 (8,1) | 2,7 (3,0) | 7,3 (7,6) | 14 (0) | | |
| 7 | $C_{34}H_{40}NO_3Cl$ | 74,5 (74,8) | 7,3 (7,3) | 2,8 (2,6) | 6,6 (6,5) | 1 (0) | 109 | in $CDCl_3$<br>$\delta = 3,6$-4,1 (m, 12H, 3C₂H₄)<br>$\delta = 4,5$ (s, 6H, 3ØCH₂O)<br>$\delta = 5,1$ (s, 2H, ØCH₂N)<br>$\delta = 7,2$-7,4 (m, 2OH, Ø) |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 8 | 236 g Tetrahydroxi-ethylethylendi-amin (1 mol)<br>784,9 g Benzylchlorid (6,2 mol)<br>240 g NaOH | 110 | 24 | 772 | $(\emptyset CH_2OC_2H_4)\overset{\oplus}{N}C_2H_4$<br>$\mid$<br>$CH_2$<br>$\emptyset$<br>      $\overset{\oplus}{N}(C_2H_4OCH_2\emptyset)_2$<br>      $\mid$<br>      $CH_2$<br>      $\emptyset$    $2\,Cl^{\ominus}$ |
| 9 | 131,1 g Dimethylneo-pentanolamin<br>265,9 g Benzylchlorid (2,1 mol)<br>80 g NaOH | 110 | 12 | 188 |             $CH_3$<br>            $\mid$<br>$(H_3C)_2\overset{\oplus}{N}CH_2-C-CH_2OCH_2\emptyset$<br>      $\mid$   $\mid$<br>      $CH_2$  $CH_3$  $Cl^{\ominus}$<br>      $\emptyset$ |
| 10 | 133 g N,N-Dimethyl-O-hydroxiethyl-ethanolamin<br>265,9 g Benzylchlorid<br>80 g NaOH | 100 | 12 | 347 | $(H_3C)_2\overset{\oplus}{N}(C_2H_4O)_2CH_2\emptyset \; Cl^{\ominus}$<br>      $\mid$<br>      $CH_2$<br>      $\emptyset$ |
| 11 | 147 g N,N-Dimethyl-O-hydroxiethyl-isopropanolamin<br>265,9 g Benzylchlorid<br>80 g NaOH | 100 | 12 | 319 | $(H_3C)_2\overset{\oplus}{N}-CH_2-CH-OC_2H_4OCH_2\emptyset$<br>      $\mid$    $\mid$<br>      $CH_2$  $CH_3$  $Cl^{\ominus}$<br>      $\emptyset$ |
| 12 | 115,2 g 1-(2-Hydroxi-ethyl)pyrrolidin<br>265,9 g Benzylchlorid<br>80 g NaOH | 100 | 12 | 235 | $CH_2-CH_2$<br>$\mid$       $\overset{\oplus}{N}C_2H_4OCH_2\emptyset \; Cl^{\ominus}$<br>        $\mid$<br>$CH_2-CH_2$  $CH_2$<br>          $\emptyset$ |
| 13 | 129,2 g 1-(2-Hydroxi-ethyl)piperidin<br>265,9 g Benzylchlorid<br>80 g NaOH | 100 | 12 | 389 |      $C_2H_4$<br>$CH_2$      $\overset{\oplus}{N}C_2H_4OCH_2\emptyset \; Cl^{\ominus}$<br>     $C_2H_4$  $\mid$<br>          $CH_2$<br>          $\emptyset$ |

| Bei-spiel Nr. | Produkt | | | | | | | |
|---|---|---|---|---|---|---|---|---|
| | Summenformel | Elementaranalyse | | | | OHZ | Smp. (EE) (°C) | $^1$H·NMR (Angaben in ppm) |
| | | C (%) | H (%) | N (%) | Cl$^\ominus$ (%) | | | |
| 8 | $C_{52}H_{62}N_2O_4Cl_2$ | 74,0 (73,5) | 7,1 (7,3) | 3,3 (3,3) | | 16 (0) | 129 | in CDCl$_3$<br>$\delta = 3,5\text{-}4,3$ (m, 20H, 10CH$_2$)<br>$\delta = 4,6$ (s, 8H, 4ØCH$_2$O)<br>$\delta = 5,3$ (s, 4H, 2ØCH$_2$N)<br>$\delta = 7,2\text{-}7,8$ (m, 30H, Ø) |
| 9 | $C_{21}H_{30}NOCl$ | 75,5 (75,6) | 8,9 (9,0) | 4,0 (4,2) | 10,0 (10,6) | 20 (0) | 94 | in CDCl$_3$<br>$\delta = 1,3$ (s, 6H, 2CH$_3$)<br>$\delta = 3,3$ (s, 6H, 2N−CH$_3$)<br>$\delta = 3,4$(s, 2H, O−CH$_2$)<br>$\delta = 3,8$ (s, 2H, N−CH$_2$)<br>$\delta = 4,5$ (s, 2H, Ø−CH$_2$O)<br>$\delta = 5,1$ (s, 2H, Ø−CH$_2$N)<br>$\delta = 7,1\text{-}7,7$ (m, 10H, Ø) |
| 10 | $C_{20}H_{28}NO_2Cl$ | 68,5 (68,6) | 8,1 (8,0) | 3,6 (4,0) | 8,4 (10,1) | 4 (0) | | in CDCl$_3$<br>$\delta = 3,3$ (s, 6H, 2CH$_3$)<br>$\delta = 3,7$ (Cl, 8H, 4CH$_2$)<br>$\delta = 4,5$ (s, 2H, Ø−CH$_2$−O)<br>$\delta = 5,0$ (s, 2H, Ø−CH$_2$−N)<br>$\delta = 7,2\text{-}7,7$ (m, 10H, Ø) |
| 11 | $C_{21}H_{30}NO_2Cl$ | 69,7 (69,3) | 8,4 (8,3) | 3,6 (3,9) | 7,8 (9,8) | 22 (0) | | |
| 12 | $C_{20}H_{26}NOCl$ | 71,9 (72,4) | 7,9 (7,8) | 3,9 (4,2) | 10,4 (10,7) | 12 (0) | 114 | in CDCl$_3$<br>$\delta = 2,2$ (m, 4H, 2CH$_2$)<br>$\delta = 3,8$ (m, 8H, 4CH$_2$)<br>$\delta = 4,6$ (s, 2H, Ø−CH$_2$O)<br>$\delta = 4,9$ (s, 2H, ØCH$_2$−N)<br>$\delta = 7,2\text{-}7,7$ (m, 10H, Ø) |
| ·13 | $C_{21}H_{28}NOCl$ | 72,5 (72,9) | 8,1 (8,1) | 3,9 (4,1) | 10,1 (10,2) | 18 (0) | 162 | in CDCl$_3$<br>$\delta = 1,8$ (m, 6H, 3CH$_2$)<br>$\delta = 3,8$ (m, 8H, 4CH$_2$)<br>$\delta = 4,6$ (s, 2H, Ø−CH$_2$−O)<br>$\delta = 5,1$ (s, 2H, Ø−CH$_2$N)<br>$\delta = 7,2\text{-}7,8$ (m, 10H, Ø) |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 14 | 270,3 g N,N-Dibutyl-N-triethylenglykol-amin<br>265,9 g Benzylchlorid<br>80 g NaOH | 100 | 12 | 464 | $(H_9C_4)_2\overset{\oplus}{\underset{\underset{\text{Ø}}{\overset{\mid}{CH_2}}}{N}}(C_2H_4O)_3CH_2\text{Ø Cl}^\ominus$ |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 15 | 175,9 g Pentahydroxi-ethyldipropylen-triamin<br>525,4 g Benzylchlorid (4,15 mol)<br>120 g NaOH | 100 | 20 | 571 | $(\emptyset CH_2OC_2H_4)_2\overset{\oplus}{N}[C_3H_6-$<br>$\underset{\emptyset}{\overset{\mid}{CH_2}}$<br>$-\overset{\oplus}{N}(C_2H_4OCH_2\emptyset)]_2C_2H_4OCH_2\emptyset$<br>$\underset{\emptyset}{\overset{\mid}{CH_2}}$    $3\ Cl^{\ominus}$ |
| 16 | 103,2 g 3-Dimethyl-aminopropanol<br>265,9 g Benzylchlorid<br>80 g NaOH | 100 | 12 | 252 | $(H_3C)_2\overset{\oplus}{N}C_3H_6OCH_2\emptyset\ Cl^{\ominus}$<br>$\underset{\emptyset}{\overset{\mid}{CH_2}}$ |
| 17 | 146,3 g Tetrahydroxi-ethylhexa-methylendiamin<br>392,5 g Benzylchlorid<br>120 g NaOH | 110 | 24 | 412 | $(\emptyset CH_2OC_2H_4)_2\overset{\oplus}{N}C_6H_{12}-$<br>$\underset{\emptyset}{\overset{\mid}{CH_2}}$ $\overset{\oplus}{N}(C_2H_4OCH_2\emptyset)_2$<br>$\underset{\emptyset}{\overset{\mid}{CH_2}}$    $2\ Cl^{\ominus}$ |
| 18 | 138,4 g Heptahydroxi-ethyltetrapro-pylenpentamin<br>395,6 g Benzylchlorid<br>120 g NaOH | 110 | 24 | 399 | $(\emptyset CH_2OC_2H_4)_2\overset{\oplus}{N}[C_2H_6-$<br>$\underset{\emptyset}{\overset{\mid}{CH_2}}$<br>$-\overset{\oplus}{N}(C_2H_4OCH_2\emptyset)_4]_4C_2H_4OCH_2\emptyset$<br>$\underset{\emptyset}{\overset{\mid}{CH_2}}$    $5\ Cl^{\ominus}$ |
| 19 | 191,3 g Triisoproanol-amin<br>519,0 g Benzylchlorid<br>160,0 g NaOH | 120 | 24 | 555 | $\emptyset CH_2\overset{\oplus}{N}(CH_2CHOCH_2\emptyset)_3$<br>$Cl^{\ominus}$    $CH_3$ |

| Bei-spiel Nr. | Produkt | | | | | | | |
|---|---|---|---|---|---|---|---|---|
| | Summenformel | Elementaranalyse | | | | OHZ | Smp. (EE) (°C) | ¹H·NMR (Angaben in ppm) |
| | | C (%) | H (%) | N (%) | $Cl^{\ominus}$ (%) | | | |
| 14 | $C_{28}H_{42}NO_3Cl$ | 70,3 (70,7) | 9,1 (8,8) | 2,5 (2,9) | 6,4 (7,5) | 15 (0) | | in CDCl₃<br>$\delta = 0,8\text{-}2,1$ (m, 14H, 2C₃H₇)<br>$\delta = 3,2$ (m, 4H, 2CH₂)<br>$\delta = 3,9$ (m, 12H, 6CH₂)<br>$\delta = 4,5$ (s, 2H, ØCH₂O)<br>$\delta = 4,9$ (s, 2H, ØCH₂N)<br>$\delta = 7,2\text{-}7,7$ (m, 10H, Ø) |

| Bei-spiel Nr. | Produkt | | | | | | | |
|---|---|---|---|---|---|---|---|---|
| | Summenformel | Elementaranalyse | | | | OHZ | Smp. (EE) (°C) | $^1$H·NMR (Angaben in ppm) |
| | | C (%) | H (%) | N (%) | $Cl^\ominus$ (%) | | | |
| 15 | $C_{72}H_{88}N_{3/5}Cl_3$ | 73,1 (73,2) | 7,9 (7,5) | 3,5 (3,6) | 6,4 (0) | 19 | | |
| 16 | $C_{19}H_{26}NOCl$ | 70,9 (71,4) | 8,1 (8,1) | 4,0 (4,4) | 11,4 (11,1) | 19 (0) | 112 | in $CDCl_3$<br>$\delta = 2,2$ (m, 2H, $CH_2$)<br>$\delta = 3,3$ (s, 6H, $2CH_3$)<br>$\delta = 3,6$ (m, 4H, $2CH_2$)<br>$\delta = 4,4$ (s, 2H, $ØCH_2O$)<br>$\delta = 4,9$ (s, 2H, $ØCH_2N$)<br>$\delta = 7,1\text{-}7,7$ (m, 10H, Ø) |
| 17 | $C_{56}H_{70}N_2O_4Cl_2$ | 75,2 (75,4) | 8,0 (7,9) | 3,0 (3,1) | 7,8 (8,0) | 18 (0) | 129 | in $CDCl_3$<br>$\delta = 1,2$ (m, 4H, $2CH_2$)<br>$\delta = 1,9$ (m, 4H, $2CH_2$)<br>$\delta = 3,2\text{-}4,2$ (m, 20H, $10CH_2$)<br>$\delta = 4,5$ (s, 8H, $4ØCH_2O$)<br>$\delta = 4,9$ (s, 4H, $2ØCH_2N$)<br>$\delta = 7,0\text{-}7,8$ (m, 30H, Ø) |
| 18 | $C_{110}H_{36}N_5O_7Cl_5$ | 72,6 (72,7) | 7,7 (7,5) | 4,0 (3,9) | 5,4 (5,6) | 16 (0) | | |
| 19 | $C_{37}H_{46}NO_3Cl$ | 75,2 (75,6) | 7,9 (7,8) | 2,3 (2,4) | 6,2 (6,0) | 10 (0) | 143 | in $CDCl_3$<br>$\delta = 1,0$ (m, 9H, $3CH_3$)<br>$\delta = 3,4$ (m, 6H, $3CH_2$)<br>$\delta = 4,5$ (m, 6H, $3ØCH_2O$)<br>$\delta = 4,6$ (s, 2H, $ØCH_2N$)<br>$\delta = 5,1$ (q, 3H, 3CH)<br>$\delta = 6,9\text{-}7,8$ (m, 20H, Ø) |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 20 | 119,2 g Methyldi-ethanolamin<br>265,9 g Benzylchlorid<br>80 g NaOH | 100 | 12 | 222 | $C_2H_4OH$<br>$\overset{\oplus}{\underset{\underset{Ø}{CH_2}}{H_3C-N-C_2H_4OCH_2Ø}}$ $Cl^\ominus$ |
| 21 | 103,2 g Dimethylisopro-panolamin<br>265,9 g Benzylchlorid<br>80 g NaOH | 100 | 12 | 317 | $(H_3C)_2\overset{\oplus}{\underset{\underset{Ø}{CH_2}}{N}}-CH_2-\underset{\underset{Cl^\ominus}{CH_3}}{CHOCH_2Ø}$ |

| Bei-spiel Nr. | Einsatzstoffe | Reaktions-bedingungen | | Aus-waage (g) | Strukturformel |
|---|---|---|---|---|---|
| | | Quater-nierungs-temperatur (°C) | Nach-rührzeit (h) | | |
| 22 | 187,9 g N-Tridecyl-N,N-bis-(O-hydroxi-ethylethanol-amin) 196,2 g Benzylchlorid 60 g NaOH | 110 | 24 | 313 | $H_{27}C_{13}\overset{\oplus}{N}(C_2H_4OC_2H_4OCH_2\emptyset)_2\ Cl^{\ominus}$ <br> $\quad\quad\mid$ <br> $\quad\quad CH_2$ <br> $\quad\quad\emptyset$ |
| 23 | 187,2 g Phenyldiethanol-amin (1 mol) 392,5 g Benzylchlorid (3,1 mol) 120 g NaOH | 110 | 24 | 445 | $\emptyset\overset{\oplus}{N}(C_2H_4OCH_2\emptyset)_2$ <br> $\mid$ <br> $CH_2 \quad Cl^{\ominus}$ <br> $\mid$ <br> $\emptyset$ |

| Bei-spiel Nr. | Produkt | | | | | | | |
|---|---|---|---|---|---|---|---|---|
| | Summenformel | Elementaranalyse | | | | OHZ | Smp. (EE) (°C) | ¹H·NMR (Angaben in ppm) |
| | | C (%) | H (%) | N (%) | $Cl^{\ominus}$ (%) | | | |
| 20 | $C_{15}H_{26}NO_2Cl$ | 68,5 (68,0) | 7,9 (7,7) | 4,4 (4,2) | 10,8 (10,6) | 176 (167) | | in $CDCl_3$ <br> $\delta = 1,9$ (s, 1H, OH) <br> $\delta = 3,2$ (s, 3H, 1$CH_3$) <br> $\delta = 3,4\text{-}4,2$ (m, 8H, 4$CH_2$) <br> $\delta = 4,5$ (s, 2H, $\emptyset-CH_2O$) <br> $\delta = 4,9$ (s, 2H, $\emptyset-CH_2N$) <br> $\delta = 7,1\text{-}7,7$ (m, 10H, $\emptyset$) |
| 21 | $C_{19}H_{26}NOCl$ | 70,9 (71,4) | 8,0 (8,1) | 4,1 (4,4) | 10,6 (11,1) | 12 (0) | | |
| 22 | $C_{42}H_{64}NO_4Cl$ | 74,5 (74,0) | 9,4 (9,4) | 2,0 (2,1) | 4,8 (5,2) | 13 (0) | | |
| 23 | $C_{31}H_{33}NO_2Cl$ | 76,3 (76,3) | 7,2 (7,0) | 3,0 (2,9) | 7,3 (7,3) | 25 | | in $CDCl_3$ <br> $\delta = 3,6$ (s, 8H, 2$C_2H_4$) <br> $\delta = 4,5$ (s, 4H, 2$\emptyset CH_2O$) <br> $\delta = 4,55$ (s, 2H, 1$\emptyset CH_2N$) <br> $\delta = 6,5\text{-}7,5$ (m, 15H, $\emptyset$) |

## Patentansprüche

1. Quaternäre Ammoniumsalze der allgemeinen Formel

$$[R^1R^2R^3N(A-O)_n-R^3]^+Z^-,$$

wobei $R^1$ und $R^2$ jeweils gleiche oder verschiedene Substituenten aus der Gruppe der Alkyl-, Aryl-, Aralkyl-, Alkylaryl-, Alkoxyalkyl- oder (Poly)-alkylenglykolreste bedeuten oder zusammen mit dem Stickstoffatom einen entsprechenden heterocyclischen Ring bilden oder wobei mindestens ein Substituent $R^1$ oder $R^2$ mehrfunktionell derart ist, dass ein wenigstens zwei Ammoniumgruppen aufweisendes Salz dargestellt wird; wobei $R^3$ einen Benzylrest, A einen Alkylenrest mit 2 bis 10 C-Atomen, n eine ganze Zahl von 1 oder mehr und Z entweder den Substituenten Chlor oder ein anderes salzbildendes Anion oder ein Hydroxylion bedeuten.

2. Verfahren zur Herstellung der Verbindungen nach Anspruch 1, dadurch gekennzeichnet, dass man ein tertiäres Alkanolamin der allgemeinen Formel $R^1R^2N(-A-O)_nH$ mit wenigstens einer Hydroxylgruppe je Molekül mit Benzylchlorid und in Gegenwart eines Phasentransferkatalysators behandelt.

## Claims

1. A quaternary ammonium salt of the general formula

$$[R^1R^2R^3N(A-O)_n-R^3]^+Z^-,$$

where $R^1$ and $R^2$ are identical or different and each is alkyl, aryl, aralkyl, alkylaryl, alkoxyalkyl or a (poly)alkylene glycol radical, or $R^1$ and $R^2$, together with the nitrogen atom, are a heterocyclic ring, or at least one of the substituents $R^1$ and $R^2$ is polyfunctional so that the salt contains not less than two ammonium groups, the $R^3$'s are each benzyl, A is alkylene of 2 to 10 carbon atoms, n is an integer of 1 or more and Z is either the substituent chlorine or another salt-forming anion or a hydroxyl ion.

2. A process for the preparation of a compound as claimed in claim 1, wherein a tertiary alkanolamine of the general formula $R^1R^2N(-A-O)_nH$ with not less than one hydroxyl group per molecule is treated with benzyl chloride in the presence of a phase transfer catalyst.

## Revendications

1. Sels d'ammonium quaternaires de formule générale

$$[R^1R^2R^3N(A-O)_n-R^3]^+Z^-,$$

dans laquelle $R^1$ et $R^2$ représentent des substituants, semblables ou différents, choisis dans le groupe des radicaux alcoyle, aryle, aralcoyle, alcoylaryle, alcoxyalcoyle ou (poly)alcoylèneglycol ou forment ensemble, avec l'atome d'azote, un noyau hétérocyclique correspondant, ou dans laquelle l'un au moins des substituants $R^1$ ou $R^2$ est polyfonctionnel, de telle manière qu'il soit formé un sel comportant au moins deux groupes ammonium, $R^3$ étant un radical benzyle, A un radical alcoylène à 2 à 10 atomes de C, n un nombre entier de 1 ou plus et Z étant le substituant chlore, un autre anion formateur de sel ou un ion hydroxyle.

2. Procédé pour la préparation des composés selon la revendication 1, caractérisé en ce qu'on traite par le chlorure de benzyle et en présence d'un catalyseur de transfert de phases une alcanolamine tertiaire de formule générale $R^1R^2N(-A-O)_nH$ comportant au moins un groupe hydroxyle par molécule.